# EUROPÄISCHE PATENTSCHRIFT

(11) **EP 0 959 138 B1**
(45) Veröffentlichungstag und Bekanntmachung des Hinweises auf die Patenterteilung: **29.09.2004**
(21) Anmeldenummer: 99109604.1
(22) Anmeldetag: 14.05.1999
(51) Int. Cl.: C12P 17/02

(54) **Verfahren zur Herstellung von Baccatin-III durch enzymatische Synthese**
Process for the preparation of Baccatin-III by enzymatic synthesis
Procédé de préparation de Baccatine-III par voie enzymatique

(30) Priorität: 22.05.1998 DE 19822881
(43) Veröffentlichungstag der Anmeldung: 24.11.1999
(73) Patentinhaber: Institut für Bioanalytik, Umwelt-Toxilogie und Biotechnologie Halle GmbH, 06120 Halle (DE)
(72) Erfinder: Glund, Konrad Dr., 06122 Halle (DE); Hoffmann, Matthias, 06667 Weissenfels (DE); Weckwerth, Wolfram, 14089 Berlin (DE); Zocher, Rainer Dr., 12169 Berlin (DE)
(74) Vertreter: Wablat, Wolfgang, Dr. Dr.

(56) Entgegenhaltungen:
- US-A- 4 261 829
- CHEMICAL ABSTRACTS, vol. 126, no. 3, 20. Januar 1997 (1997-01-20) Columbus, Ohio, US; abstract no. 29184, ZOCHER, RAINER ET AL: "Biosynthesis of taxol: enzymic acetylation of 10 deacetylbaccatin-III to baccatin-III in crude extracts from roots of Taxu baccata" XP002113203 & BIOCHEM. BIOPHYS. RES. COMMUN. (1996), 229(1), 16-20 ,
- PENNINGTON, J. J. ET AL.: "Acetyl CoA: 10-Deacetylbaccatin-III-10-O-acetyltransfe rase Activity in Leaves and Cell Suspension Cultures of Taxus Cuspidata." PHYTOCHEMISTRY, Bd. 49, Nr. 8, 1998, Seiten 2261-2266, XP002113202

## Beschreibung

Die Erfindung betrifft ein neues Verfahren zur kontinuierlichen Herstellung von Baccatin III mittels enzymatischer Acetylierung von 10-Deacetylbaccatin-III (10-DAB) in einem Enzymreaktor.

10-DAB und Baccatin III sind Intermediate bei der Biosynthese von Taxol und können in unterschiedlichen Konzentrationen aus Eiben (Taxus spec.) isoliert werden (0,0002 % Baccatin III aus der Rinde von Taxus baccata, 0,02 % 10-DAB aus Nadeln und Zweigen, Kingston, D.G.I, Pharm. Ther., 1991, 52, 1-34).

Taxane, speziell Taxol (Paclitaxel) sind cytotoxische Diterpene aus der Eibe, von denen einige molekular die Zellreplikation hemmen, indem sie wachsende Zellen in der G2-/M-Phase des Zellzyklus inhibieren. Sie haben daher eine Anti-Tumor-Wirkung und werden zunehmend gegen eine Reihe von Carzinomen (Eierstock-, Brust-, Bronchial- und Lungen-Carzinoma) eingesetzt.

Taxane und verwandte Wirkstoffe werden von Pflanzen der Gattung Taxus erzeugt und sind Inhaltsstoffe verschiedener Teile solcher Pflanzen. Es ist darum eine technisch und ökonomisch sinnvolle Aufgabenstellung, die Voraussetzungen für eine rationelle Gewinnung von Taxanen (speziell Taxol) aus Taxus-Pflanzen zu entwickeln und daraus Methoden zur Gewinnung dieses Stoffes und seiner Analoga abzuleiten.

Die zur Zeit einzig genutzte Quelle für die Gewinnung von Taxol in größerem Maßstab ist die Aufarbeitung der Rinde von Taxus brevifolia Nutt. Hochrechnungen besagen, dass etwa 9 t Rinde für die Produktion von 1 kg Taxol aufgearbeitet werden müßten.

Da dieses Verfahren absehbar zur Zerstörung des Eibenbestandes führen würde, wurden Alternativen der Gewinnung durch chemischen Voll- bzw. Semisynthesen (Holton. R.A. and Ojima, I; EP-A 400971, 1990) gesucht.

Die erstgenannte Möglichkeit der Gewinnung durch Vollsynthese hat sich aber auf Grund ihrer Kompliziertheit absehbar als nicht geeignet erwiesen, den bereits bestehenden Bedarf bei vertretbarem Aufwand zu decken.

Eine erfolgreichere Methode ist die Semisynthese, ausgehend von 10-DAB 10-DAB ist in etwa 6 bis 10facher Konzentration in den Nadeln der Europäischen Eibe (Taxus baccata) als Taxol in der Rinde von Taxus brevifolia Nutt. enthalten, kann also in größerer Menge aus einer nachwachsenden Rohstoffquelle extrahiert werden.

Die Reinigung von 10-DAB ist wesentlich einfacher und ökonomischer als die Gewinnung von Taxol, die aufwendige Abtrennung des strukturell sehr ähnlichen Cephalomannin entfällt. Während Taxol in hochreiner Form für die direkte Arzneimittel-Formulierung bereitzustellen ist, muss die Reinheit von 10-DAB nur ausreichend für die Nutzung als Ausgangsstoff der Semisynthese sein. Mit 10-DAB geht man außerdem von dem Startmolekül aus, dessen Herstellung durch Vollsynthese nur mit einem ökonomisch kaum vertretbar hohen Aufwand möglich ist.

Die chemische Synthese von Baccatin-III wird erfolgreich durch spezifische Acetylierung der Hydroxylgruppe an Position 10 von 10-DAB durchgeführt.

Nachteilig wirkt sich aber aus, dass die chemische Acetylierung des Substrates unspezifisch ist. Für die vier Hydroxylgruppen im 10-DAB wurden unterschiedliche Reaktivitäten gegenüber einer Acetylierung nachgewiesen. Die reaktivste ist die C-7 Hydroxylgruppe, gefolgt von den Hydroxylgruppen an Position C-10 und Position C-13. Die C-1 Hydroxylgruppe hingegen wurde unter keiner der untersuchten Bedingungen acetyliert. Unerwünschte Acetylierungen an C-7 und C-13 müssen durch entsprechend aufwendige chemische Blockierung der Hydroxylgruppen verhindert werden. Nach erfolgreicher Acetylierung an C-10 müssen die Blocker wieder entfernt werden.

Es bestand deshalb die Aufgabe, die enzymatische Acetylierung von 10-DAB mit Zell-Rohextrakten aus den Wurzeln von Eiben (Taxus spec.) erfolgreich durchzuführen.

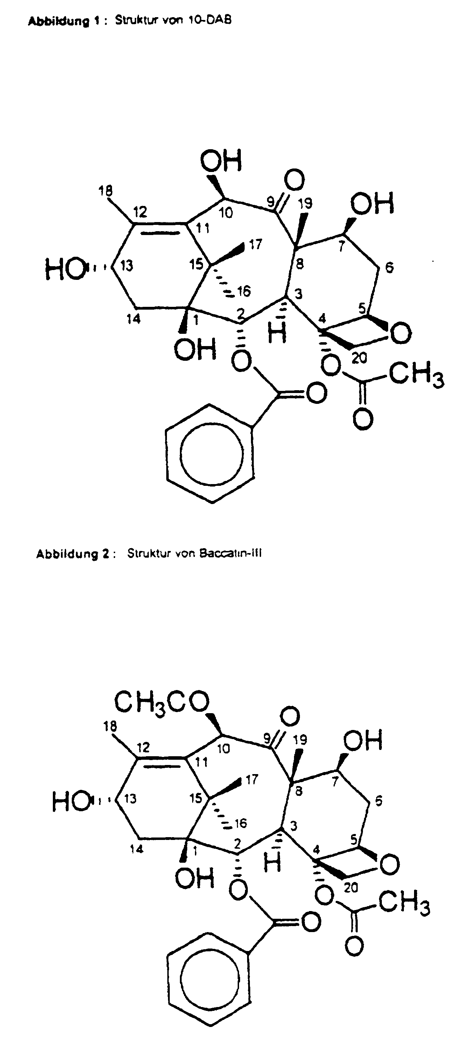

Grundsätzlich ist der Reaktionsmechanismus der aus Taxus baccata isolierten Acetyl-Transferase bekannt (Zocher, R., Weckwerth, W., Hacker, C., Kammer, B., Hornbogen, T., Ewald, D.; B.B.R.C., 1996, 229, 16-20), d. h.
1. Die Umwandlung von 10-DAB in Baccatin III ist außerhalb des Organismus (in vitro) möglich.
2. Die Acetyl-Transferase aus Taxus baccata ist hochspezifisch, d. h. es wird ausschließlich die OH-Gruppe an Position C-10 des Substrates 10-DAB acetyliert (Strukturen siehe Abbildungen 1 und 2).

Einer rationellen technischen Anwendung der Nutzung stehen jedoch einige Probleme entgegen:
- Bei einem enzymatischen Batch-Verfahren geht das eingesetzte Enzym bei der Aufarbeitung verloren.
- Die Operationszeit im Batch-Verfahren liegt im Stundenbereich.
- Im Batch-Verfahren treten bei längeren Inkubationszeiten Kontaminationen durch Mikroorganismen auf.
- Eine Immobilisierung zur Verbesserung der technischen Eigenschaften führt immer zu starken Aktivitätsverlusten.

Es bestand deshalb die Aufgabe, ein technisch nutzbares Verfahren zur enzymatischen Synthese von Baccatin III durch Acetylierung von 10-DAB zu entwickeln, das diese Nachteile vermeidet.

Diese Aufgabe wird durch ein Verfahren gelöst, bei dem ein wässriger Reaktionsansatz verwendet wird, der eine partiell gereinigte oder hochreine Acetyl-Transferase, wie die im Taxus spec. enthaltene, und als Synthesekomponenten 10-DAB, Acetyl-Coenzym A und ein Acetyl-Coenzym A regenerierendes System sowie ein Schutzkolloid gelöst enthält, und der über eine semipermeable Membran von einem organischen Lösungsmittel getrennt ist, das als Extraktionsmittel für Baccatin-III dient, wobei sowohl der wässrige Reaktionsansatz regelmäßig in einem wässrigen Puffersystem regeneriert als auch das Lösungsmittel kontinuierlich oder in regelmäßigen Abständen gewechselt wird.

Überraschend wurde festgestellt, dass die in Taxus baccata enthaltene Acetyl-Transferase auch in Gegenwart organischer Lösungsmittel stabil ist und dass sich die Operationsstabilität des Enzyms drastisch erhöht, wenn der sich in einer semipermeablen Membran befindliche wässerige Reaktionsansatz von einem geeigneten organischen Lösungsmittel umgeben ist und in definierten Zeiten durch Dialyse im wässrigen Puffersystem regeneriert wird. Die Anwesenheit des organischen Lösungsmittels verhindert eine mikrobielle Kontamination.

Eine Vorzugsvariante besteht darin, dass dem wässrigen Reaktionsansatz eine Puffersubstanz zugesetzt wird.

Die Verfahrensweise sieht
- die enzymatische Acetylierung von 10-DAB zu Baccatin III durch die Acetyl-Transferase,
- die Extraktion des im wässrigen Ansatz durch enzymatische Katalyse entstandenen Baccatin III durch ein geeignetes organisches Lösungsmittel,
- den kontinuierlichen Ersatz des mit Baccatin III angereicherten Lösungsmittels und damit eine kontinuierliche Produktabführung und
- eine Regeneration des Reaktionsansatzes durch Dialyse in einem wässrigen Puffersystem vor.
- eine Regeneration des Reaktionsansatzes durch Dialyse in einem wässrigen Puffersystem vor.

Neben der Acetyl-Transferase sollte der Reaktionsansatz vorzugsweise ein Enzym zur Regeneration von Acetyl-Coenzym A (CoA) enthalten, das kurzkettige Fettsäuren (Essigsäure, Propionsäure oder Acrylsäure) aktiviert, d. h. in den Acyl-CoA-Thioester überführt.

Das Acetyl-Coenzym A (CoA) regenerierende System besteht aus:
- dem Coenzym A
- der Acetyl-Coenzym A-Synthetase oder -Ligase
- Acetat
- einer Energiequelle - und zwar Adenosin-5-Triphosphat (ATP), da bei der Fettsäureaktivierung Energie verbraucht wird.

Das vorliegende Verfahren erlaubt den halbkontinuierlichen oder kontinuierlichen Einsatz der Acetyl-Transferasen zur Herstellung von Baccatin III.

Das Verfahren ist gut geeignet, unter Verwendung von isotopenmarkiertem Acetyl-CoA radioaktiv markiertes Baccatin-III zu synthetisieren.

Die Vorzugsvariante besteht darin, Acetyl-Transferasen aus Eiben (Taxus spec.) zu verwenden.

Vorteilhaft ist es auch, wenn als Acetyl-Transferase eine überexprimierte, rekombinante Acetyl-Transferase verwendet wird.

Eine überexprimierte, rekombinante Acetyl-Transferase ist das im Reaktionsansatz verwendete Enzym, das mittels gentechnischer Methoden in einem anderen Organismus als Taxus spec., z.B. in einem Pilz oder einer Hefe, hergestellt wurde.

Die Dialyse- oder semipermeable Membran ist für die Reaktionsprodukte durchlässig, nicht jedoch für das Enyzm. Vorzugsweise besteht sie aus Cellulose-Ester.

Die Dialyse- oder semipermeable Membran ist von einem geeigneten organischen Lösungsmittel umgeben. Geeignet sind alle Lösungsmittel, in denen das Produkt löslich ist und die mit Wasser Zweiphasensysteme bilden.

Vorzugsweise werden als organisches Lösungsmittel Chloroform und Heptan verwendet. Besonders geeignet ist ein Chloroform-Heptan-Gemisch im Verhältnis 1 : 4.

Die Vorteile des erfindungsgemäßen Verfahrens gegenüber der chemischen Acetylierung, die zur Zeit technisch angewandt wird, liegen in der Spezifität der Acetylierung. Durch geeignete Reaktionsführung in einem Enzymreaktor kann das Produkt kontinuierlich aus dem Reaktionsansatz entfernt werden und direkt zur Semisynthese des Taxols und Taxoters eingesetzt werden. Dadurch werden die Operationszeit (mindestens Wochen) und die erzielbaren Ausbeuten (mg-g Produkt pro mg eingesetztes Enzym) wesentlich erhöht.

Das Verfahren soll anhand des folgenden Beispiels erläutert werden, ohne darauf beschränkt zu sein.

### Ausführungsbeispiel

Alle Operationen werden bei 4 °C durchgeführt. 10 g von zuvor bei -80 °C gelagerten Taxus baccata Wurzeln werden unter wiederholter Zugabe von flüssigem Stickstoff in einem Mörser zerkleinert. Unter Zugabe von Seesand wird das Material zu einem feinem Pulver zermörsert und anschließend in 50 mM Tris/HCL Puffer (Zusammensetzung siehe Biochem. Biophys. Res. Commun. 229, 16-20 (1996)) aufgenommen (4 ml Puffer/g Wurzeln Frischgewicht). Die Suspension wird eine Stunde vorsichtig gerührt. Nachdem die Zelltrümmer durch Zentrifugation bei 10000 rpm für 20 min abgetrennt worden sind, wird mit dem Überstand eine 30%ige Ammoniumsulfat-Fällung durchgeführt (15 min auf Eis). Das Pellet wird durch Zentrifugation (10000 rpm/20 min) abgetrennt und mit dem verbleibenden Überstand wird eine 30 bis 90%ige Ammoniumsulfat-Fällung (1 h auf Eis) durchgeführt. Nach erneuter Zentrifugation (10000 rpm/20 min) wird das Pellet vom Überstand abgetrennt und in wenig Puffer (s.o.) aufgenommen. Diese resuspendierte 30 bis 90%ige Ammoniumsulfat-Fällung wird mit einer kleinen Sephadex G 25 (Pharmacia PD 10)-Kolonne entsalzt, und mit der resultierenden Fraktion werden alle enzymatischen Reaktionen durchgeführt.

Die Synthese wird im Enzymreaktor unter folgenden Bedingungen durchgeführt:

Als Lösungsmittel kommt ein Gemisch aus Chloroform : Heptan im Verhältnis 1 : 4 zum Einsatz. Der Dialysesack enthält eine wässrige Lösung mit folgenden Komponenten:
- 1 ml Acetyl-CoA:DAB-O-Transferase (Proteinkonzentration 100 µg/ml, DEAE Fraktion) in 0,05 M HEPES-Puffer, pH 8;
- 1 % Rinderserum-Albumin,
- 50 µM 10-DAB;
- 5 % Ethanol;
- 50 µM Acetyl-CoA
- 2 µM [³H-Acetyl]-CoA

Das gebildete radioaktive Baccatin III (mit dem ³H) geht in die organische Phase über, die während des Reaktorbetriebes mehrfach gewechselt wird. Die Aufarbeitung des Reaktionsproduktes erfolgt durch RP HPLC, da ein Teil des Eduktes 10-Deacetylbaccatin III mit in die organische Phase übergeht und nach der Trennung wieder in den Prozeß zurückgeführt werden kann.

Nach 12 h Inkubation bei Raumtemperatur überführt man die Dialysemembran mit dem Enzym in ein Glasgefäß mit 0,05 M HEPES-Puffer, pH 8, worin sie 12 h verbleibt.

Der Dialyseschritt dient zur Regenerierung des Reaktionsansatzes, wobei in erster Linie entstandenes Coenzym A abgeführt wird.

Danach wird der Ansatz mit neuen Substraten versetzt und wiederholt in den Enzymreaktor eingebracht. Es wird wechselweise mit zwei Enzymportionen gearbeitet, damit die Reaktion ständig in Betrieb gehalten werden kann.

Unter den genannten Bedingungen lassen sich, auf 1 mg Acetyltransferase bezogen, ca. 1 mg Baccatin III pro Stunde produzieren.

### Zur Selektivität:

Die enzymatische Acetylierung findet spezifisch an Position 10 statt. Weitere Substrate. z. B. Taxol, Baccatin III mit freien OH-Gruppen, wurden wie im Ausführungsbeispiel eingesetzt und nicht acetyliert.
10 Deacetyltaxol, eingesetzt wie im Ausführungsbeispiel, ist ein wesentlich schwächeres Substrat als 10 Deacetylbaccatin III.

## Patentansprüche

1. Verfahren zur Herstellung von Baccatin III durch Acetylierung von 10-Deacetylbaccatin (10-DAB) durch enzymatische Synthese in einem Enzymreaktor, **dadurch gekennzeichnet, dass** ein wässriger Reaktionsansatz verwendet wird, der eine partiell gereinigte oder hochreine Acetyl-Transferase wie die in Taxus spec. enthaltene, und als Synthesekomponenten 10-DAB, Acetyl-Coenzym A und ein Acetyl-Coenzym-A-regenerierendes System, sowie ein Schutzkolloid gelöst enthält, und der über eine semipermeable Membran von einem organischen Lösungsmittel getrennt ist, das als Extraktionsmittel für Baccatin III dient, wobei sowohl der wässrige Reaktionsansatz regelmäßig in einem wässrigen Puffersystem regeneriert als auch das Lösungsmittel kontinuierlich oder in regelmäßigen Abständen gewechselt wird.

2. Verfahren nach Anspruch 1, **dadurch gekennzeichnet, dass** dem wässrigen Reaktionsansatz eine Puffersubstanz hinzugefügt wird.

3. Verfahren nach Anspruch 1 oder 2, **dadurch gekennzeichnet, dass** zur Regeneration von Acetyl-Coenzym A eine Acetyl-CoA-Ligase oder Acetyl-CoA-Synthetase zugesetzt wird.

4. Verfahren nach einem oder mehreren der Ansprüche 1 bis 3, **dadurch gekennzeichnet, dass** unter Verwendung von isotopenmarkiertem Acetyl-CoA radikoaktiv markiertes Baccatin-III synthetisiert wird.

5. Verfahren nach einem oder mehreren der Ansprüche 1 bis 4, **dadurch gekennzeichnet, dass** als Acetyl-Transferase eine überexprimierte, rekombinante Acetyl-Transferase verwendet wird.

6. Verfahren nach einem oder mehreren der Ansprüche 1 bis 5, **dadurch gekennzeichnet, dass** die semipermeable Membran aus Cellulose-Ester besteht.

7. Verfahren nach einem oder mehreren der Ansprüche 1 bis 6, **dadurch gekennzeichnet, dass** als organisches Lösungsmittel ein Chloroform-Heptan-Gemisch, vorzugsweise im Verhältnis 1 : 4, eingesetzt wird.

## Claims

1. A method for producing baccatin III by acetylating 10-deacetyl baccatin (10-DAB) by enzymatic synthesis in an enzyme reactor wherein a hydrous reaction batch is used that contains partially purified or ultrapure acetyl transferase such as the one found in taxus spec., acetyl coenzyme A or a system regenerating acetyl coenzyme A, and a protective colloid in dissolved form, separated by a semipermeable membrane from an organic solvent that serves as extraction solvent for baccatin III, said hydrous reaction batch regularly regenerating in a hydrous buffer system and said solvent being replaced continuously or at regular intervals.

2. The method according to claim 1 wherein a buffer substance is added to the hydrous reaction batch.

3. The method according to claims 1 or 2 wherein an acetyl CoA ligase or acetyl CoA synthetase is added for the regeneration of acetyl coenzyme A.

4. The method according to one or several of the preceding claims 1 through 3 wherein radiolabeled baccatin III is synthesized using isotopically labeled acetyl CoA.

5. The method according to one or several of the preceding claims 1 through 4 wherein an overexpressed recombinant acetyl transferase is used as acetyl transferase.

6. The method according to one or several of the preceding claims 1 through 5 wherein the semipermeable membrane consists of cellulose ester

7. The method according to one or several of the preceding claims 1 through 5 wherein a chloroform-heptane mixture, preferably at a 1 to 4 ratio, is used as an organic solvent.

## Revendications

1. Procédé de préparation de baccatine III par acétylation de 10-désacétyl-baccatine (10-DAB) par synthèse enzymatique dans un réacteur enzymatique, **caractérisé en ce que** l'on utilise un mélange réactionnel aqueux qui contient en solution une acétyltransférase partiellement purifiée ou de haute pureté telle que celle contenue dans *Taxus* spp. et, comme composants de synthèse, de la 10-DAB, de l'acétylcoenzyme A et un système régénérant l'acétylcoenzyme A, ainsi qu'un colloïde protecteur, et qui est séparé par une membrane semi-perméable d'un solvant organique qui sert d'agent d'extraction pour la baccatine III, le mélange réactionnel aqueux étant régulièrement régénéré dans un système de tampon aqueux, de même que le solvant est changé en continu ou à intervalles réguliers.

2. Procédé selon la revendication 1, **caractérisé en ce que** l'on ajoute au mélange réactionnel aqueux une substance tampon.

3. Procédé selon la revendication 1 ou la revendication 2, **caractérisé en ce que**, pour la régénération de l'acétylcoenzyme A, on ajoute une acétyl-CoA-ligase ou une acétyl-CoA-synthétase.

4. Procédé selon l'une ou plusieurs des revendications 1 à 3, **caractérisé en ce que** l'on synthétise de la baccatine III marquée radioactivement en utilisant de l'acétyl-CoA marqué par un isotope.

5. Procédé selon l'une ou plusieurs des revendications 1 à 4, **caractérisé en ce que** l'on utilise comme acétyltransférase une acétyltransférase recombinante surexprimée.

6. Procédé selon l'une ou plusieurs des revendications 1 à 5, **caractérisé en ce que** la membrane semi-perméable se compose d'ester de cellulose.

7. Procédé selon l'une ou plusieurs des revendications 1 à 6, **caractérisé en ce que** l'on utilise comme solvant organique un mélange de chloroforme et d'heptane, de préférence selon un ratio de 1:4.
